# EUROPEAN PATENT SPECIFICATION

(11) **EP 4 091 667 B1**
(45) Date of publication and mention of the grant of the patent: **20.12.2023**
(21) Application number: 22173343.9
(22) Date of filing: 13.05.2022
(51) Int. Cl.: A61N 5/06, A61B 18/00

(54) **HELMET FOR THE TREATMENT OF THE SCALP AND HAIR WITH PHOTO-BIOSTIMULATION TECHNOLOGY**
HELM ZUR BEHANDLUNG DER KOPFHAUT UND HAARE MIT PHOTOBIOSTIMULATIONSTECHNOLOGIE
CASQUE POUR LE TRAITEMENT DU CUIR CHEVELU ET DES CHEVEUX À L'AIDE DE LA TECHNOLOGIE DE PHOTO-BIOSTIMULATION

(30) Priority: 17.05.2021 IT 202100012587
(43) Date of publication of application: 23.11.2022
(73) Proprietor: Sanders S.r.l., 20124 Milano (IT)
(72) Inventor: ANGELINI, Matteo, London (GB)
(74) Representative: Rapisardi, Mariacristina

(56) References cited:
- KR-A- 20170 093 650
- US-A1- 2011 015 707
- US-A1- 2011 092 863
- US-A1- 2016 106 999
- US-A1- 2018 008 839

## Description

The present invention relates to a helmet for the treatment of the scalp and hair.

### Prior Art

The present invention relates in particular to the field of low-level laser therapy (LLLT) based treatment devices. It is an internationally recognized low-power laser therapy. It is also known as phototherapy or photo-biostimulation. The technique involves the use of a depowered laser, i.e. a laser with a low power setting and a diffuse defocused ray, which is capable of generating an intracellular biological response in the tissues without producing thermal effects. The light irradiation produced by the laser ray provides energy to the cells, which respond with an improvement in cell metabolism and an increase in blood circulation in the tissues.

Respective prior art is known from KR 2017 0093650 A, US 2016/106999 A1, US 2011/015707 A1, US 2011/092863 A1 and US 2018/008839 A1.

The problem of hair loss is very widespread nowadays and afflicts several people, both men and women, also affecting their quality of life and interpersonal relationships.

People with hair loss problems, both men and women, are increasing exponentially due to lifestyle, stress, pollution etc.

The most common abnormalities affecting the scalp are often associated with itchiness, flaking, dandruff and seborrhoea.

There are several cosmetic treatments on the market that aim to reduce the problems listed above.

Hair loss treatment technologies also include hair loss treatment devices that use massages or lasers.

In particular, laser treatment is a method already widely applied in the bio-medical field. It is a method for strengthening mainly the roots of the hair by providing nutrients and oxygen to the cells of the hair follicle through the expansion of the capillaries, the promotion of protein synthesis and the increase of blood flow through laser irradiation.

Inhibiting hair loss and inducing hair growth is the key to treatment.

In recent years, the technique that uses red and infrared rays has been consolidated, which have proven to be very effective in the treatment of hair loss and various devices that use them have been studied and developed. These devices use homogeneous emissions, i.e. they emit only in the red or infrared spectrum.

Recently, several helmet-shaped devices have been placed on the market to irradiate the upper shell of the head.

However, such devices often do not versatilely adapt to the rather variable dimensions and configurations of each user's head, thus resulting in an efficacy of the treatment that may from time to time significantly depend on how the helmet is positioned.

In addition, these devices are standardized and do not meet the specific needs and thinning patterns that may be different for the different users, and for example they are normally different in men and women.

The task of the present invention is therefore to provide a helmet for the treatment of the scalp and hair that allows to solve the drawbacks described in the prior art.

As part of this technical task, an aim of the present invention is to provide a helmet for the treatment of the scalp and hair that can be customized based on the specific individual needs of the user.

Another aim of the invention is to provide a helmet for the treatment of the scalp and hair that can be easily used at home by the subject having trichological problems.

Still another aim of the invention is to provide a helmet for the treatment of the scalp and hair that is fully customizable based on the fit of the helmet on the basis of the dimensions of the head of each user.

A further aim of the invention is to provide a helmet for the treatment of the scalp and hair that is fully customizable based on the hair thinning pattern.

A further aim of the invention is to provide a helmet for the treatment of the scalp and hair that can provide repeatable results as the conformational anatomy of the user's head varies.

The aims of the present invention are achieved by means of a helmet which can be worn on a user's head for the treatment of the scalp and hair with photo-stimulation technology comprising a shell having first diodes configured for the emission of light in the infrared field and second diodes configured for the emission of light in the visible field and arranged to irradiate the front, parietal, occipital temporal area, and the entire vertex of a user's head, said shell also having adjustable centring means for centring said helmet with respect to the cranial surface of the user characterized in that it has a controller programmed with a first program for the simultaneous activation of said first diodes and with a second program for the sequential activation of a part of said first diodes from a central area of the helmet to peripheral areas of the helmet corresponding to lymph drainage areas of the head.

Other salient aspects of the invention are set forth in the following dependent claims.

Further features and advantages of the invention will become more apparent from the description of a preferred but non-exclusive embodiment of the helmet for the treatment of the scalp and hair illustrated by way of illustration and not limitation in the accompanying drawings, wherein:

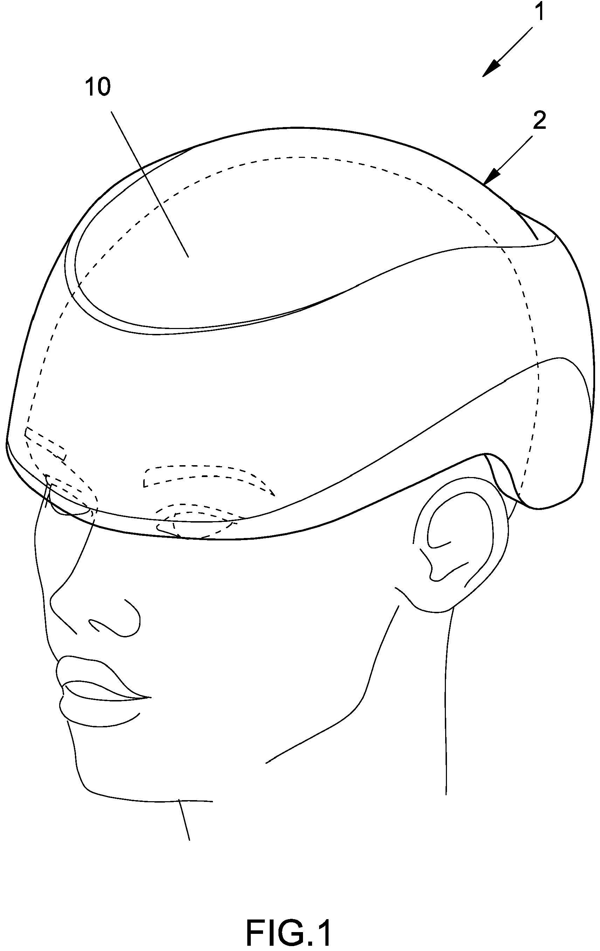

- figure 1 shows an axonometric view of the outer shell of the helmet worn by a user
- figure 2 shows a plan view from below of the inner part of the helmet
- figure 2a shows in section a detail of the helmet of figure 2;
- figure 2b shows in section a detail of the helmet's stabilizing element of figure 2;
- figure 2c shows the spacing element according to an embodiment of the invention;
- figure 3 shows a side view of the helmet in which the arrangements of the centring means and the stabilizing element are highlighted;
- figure 4 shows a plan view from below of the helmet illustrating the arrangement of the first diodes that emit infrared light and of the second diodes that emit red light based on the male thinning pattern;
- figure 5 shows the plan view of figure 4 in which arrows indicate the direction of progressive activation of a part of the first diodes from a central area of the helmet to peripheral areas of the helmet;
- figure 6 shows a plan view from below of the helmet illustrating the arrangement of the first diodes that emit infrared light and of the second diodes that emit red light based on the female thinning pattern;
- figure 7 shows the plan view of figure 6 in which arrows indicate the direction of progressive activation of a part of the first diodes from a central area of the helmet to peripheral areas of the helmet;
- figure 8 shows a plan view of the extension of the printed circuit of the diodes according to a preferred embodiment of the invention, where dots indicate the positions of the diodes;
- figures 9 and 10 each show a plan view of the extension of the printed circuit of figure 8 in which two different irradiation programs are highlighted that require the sequential activation of a different selection of the first diodes;
- figure 11 shows the positions, indicated with circled areas, of the red LEDs positioned at the front area, the temple area, the area of the entire vertex and the lateral areas of a user's head;
- figure 12 shows the positions, indicated by stars, of the red laser LEDs positioned at the front area, the temple area, the area of the entire vertex and the lateral areas of a user's head;
- figure 13 shows an exploded view of a further embodiment of the helmet.

Unlike the helmets currently commercially available in which only the upper part of the head is irradiated, the present invention advantageously includes the irradiation not only of the upper part, but also of the lower vertex and of the lateral areas of the user's head.

With reference to the aforementioned figures, a helmet for the treatment of the scalp and hair is shown, indicated as a whole with reference number 1.

The helmet 1 comprises a shell 2 formed by an outer surface 10 and by an inner surface 3.

The shell 2 has first diodes 4 configured for the emission of light in the infrared field and second diodes 5 configured for the emission of light in the visible field, in particular red light, and arranged to irradiate the front area, the temple area, the area of the entire vertex and the lateral areas of the head 23 of a user.

The outer surface 10 of the shell 2 is made of plastic material, in particular for example ABS (Acrylonitrile Butadiene Styrene). It is a tough and shock-resistant plastic, a great solution for making portable devices and is also cheap.

The inner surface 3 of the shell 2 is made of plastic material, for example transparent polycarbonate.

The shell 2 extends widthwise in the direction of the ear up to the parietal parts of the head 23 and lengthwise from the forehead to the nape of the neck so as to cover all male and female thinning areas.

Advantageously, the shell 2 has adjustable centring means 6 for centring the helmet 1 with respect to the cranial surface of the user.

According to a first embodiment of the invention, the centring means 6 comprises movable spacing elements 8 in sliding seats 7.

The spacing elements 8 have a shape and friction coupling with said sliding seats 7 to maintain the position once adjusted.

The sliding seats 7 comprise in particular grooves 15.

The grooves 15 extend in the front-rear direction of the helmet 1.

The spacing elements 8 are made of silicone material.

In particular, the spacing elements 8 are positioned in the spaces between the diodes 4 and 5.

The spacing elements 8 allow the distance to be maintained at about 2 cm between the upper part of the skull and said first diodes 4 and said second diodes 5.

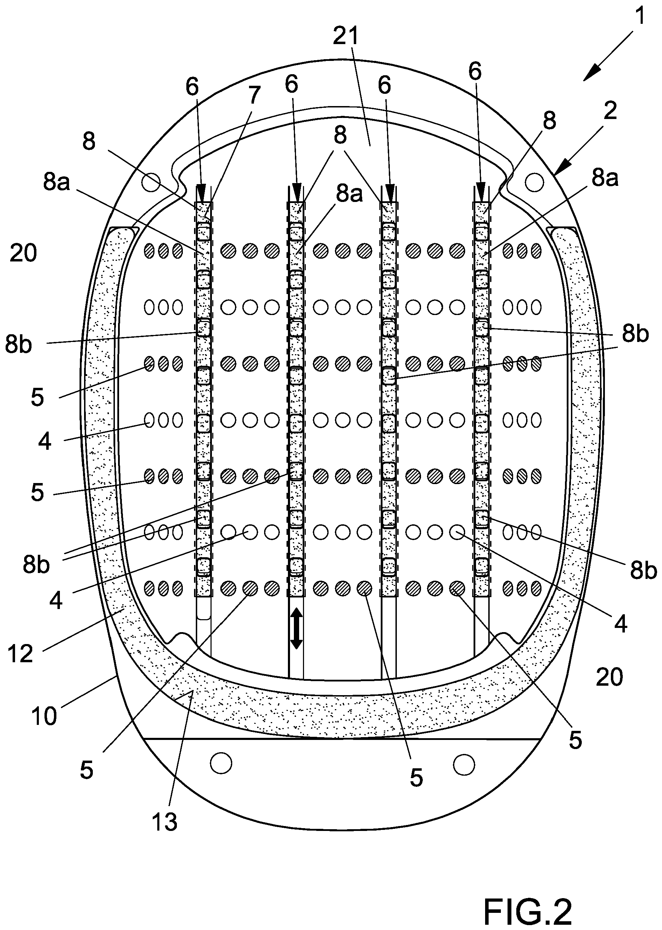

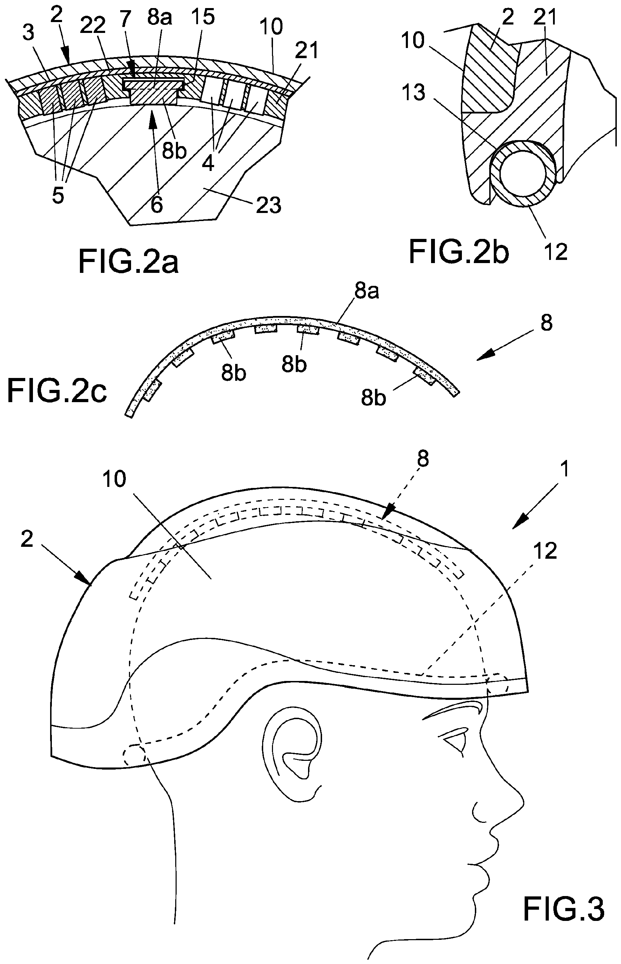

As shown in figs. 2 and 2a, the spacing elements 8 are each formed by a strip 8a having a series of lower protrusions 8b distributed at an appropriate pitch along the longitudinal axis of the strip itself.

The protrusions 8b have a large rest surface in order to effectively distribute the weight of the helmet homogeneously over the entire head 23.

Advantageously, the spacing elements 8 can be adjusted in position by hand to vary the contact area of the protrusions 8b with the user's cranial surface and thus avoid that areas of the user's cranial surface cannot be reached by the rays emitted by the diodes 4, 5.

Moreover, the possibility of moving the spacing elements 8 allows to avoid exerting pressure always on the same points of the head 23 and in this way avoids causing soreness.

The helmet 1 can be fully customized based on the specific needs of each user.

In particular, four sizes are envisaged based on the circumference of the skull: S, M, L, XL.

Customization in the 4 sizes is possible by modulating the dimensions of the spacing elements 8 and of the stabilizing elements.

Advantageously, in fact, the helmet 1 comprises an interchangeable stabilizing element 12 to stabilize the fit of the helmet 1 as the size of the user's head 23 varies.

The stabilizing element 12 is removably housed in a seat 13 positioned along an inner perimeter edge 2 of the helmet 1.

The stabilizing element 12 is made of soft material and is housed by mechanical interference inside the seat 13.

The stabilizing element 12 has a tubular conformation and is made of plastic material, in particular ABS (Acrylonitrile Butadiene Styrene) and of silicone material.

In order to customize the size of the helmet, the diameter of the stabilizing element 12 can be chosen from various measures that are all compatible for the housing by mechanical interference inside the seat 13, taking advantage of the intrinsic deformability of the stabilizing element 12.

In particular, according to the present invention, four different diameters of the stabilizing element 12 can be envisaged:
- 20mm size S
- 15mm size M
- 10mm size L
- 5mm size XL

The presence of the stabilizing element 12 contributes to constantly maintaining 2 cm of distance between the first and second diodes and the skull which are important for the correct irradiation of the user's head without tilting the helmet.

Advantageously, the first diodes 4 and the second diodes 5 are interchangeably supported to adapt the irradiation pattern in a patient-specific manner.

Preferably all the diodes 4 and diodes 5 are supported by a single support 21.

The single support 21 can be removably connected to the shell 2, in particular to the inner surface of the latter.

In particular, the single support 21 has a printed circuit 22 on which the diodes 4 and the diodes 5 are mounted.

Of course, the support 21 must have both a means for the mechanical connection to the shell 2, for example a quick coupling means, and an electrical connection means for the connection of the printed circuit 22 to the shell 2, since the electrical power supply of the diodes 4, 5 is also preferably arranged in the shell 2.

Optionally, the possibility of applying to the inner surface 3 of the shell 2 a support 21 chosen from a plurality of interchangeable supports that have different distributions of the diodes 4 and 5 is envisaged.

In this case, the sliding seats 7 of the spacing elements 8 and the seat 13 of the stabilizing element 12 can also be obtained on the support 21.

According to one aspect of the invention, the helmet 1 is a LED/LLLT hybrid in that it comprises both first diodes 4, in particular LEDs (hereinafter called infrared LEDs), which emit light in the infrared field at 905 nm, and second diodes 5, in particular LEDs (hereinafter called red LEDs) and laser LEDs (hereinafter called red laser LEDs), which emit light in the red field at 650 nm.

Therefore, the helmet 1, according to the present invention, provides 3 types of emission:
- Infrared LEDs
- Red LEDs
- Red laser LEDs

The first diodes 4 and the second diodes 5, as mentioned, are distributed within the helmet in a manner congruent with a thinning pattern.

This particular arrangement of the diodes allows further customization of the helmet based on the specific needs of the user.

In fact, men and women have different thinning patterns. In particular, in men, thinning affects the temples, the front area, the upper central area and the vertex. In women, on the other hand, thinning affects the central partition, the parietals, the upper central portion and the vertex.

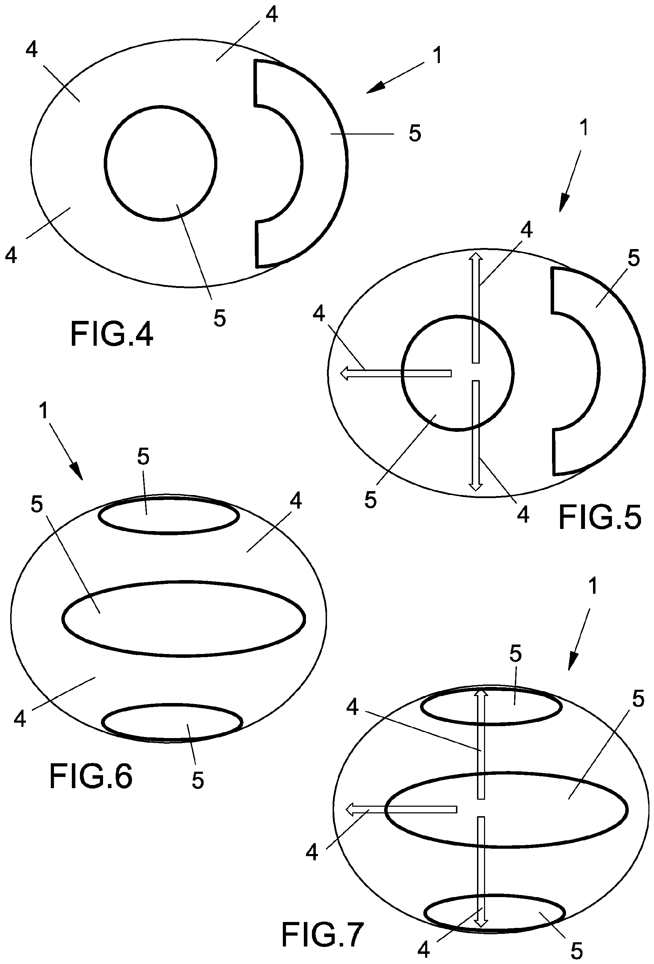

By way of not-limiting example, in the case of a male patient, as illustrated in figure 4, the second diodes 5 configured to emit light in the visible field will be positioned in the helmet 1 at the areas subject to thinning, i.e. temples, front area, upper central area and vertex.

In the case of a female patient, as illustrated in figure 6, the second diodes 5 configured to emit light in the visible field will be positioned in the helmet 1 at the areas subject to thinning, i.e. central partition, the parietals, the upper central portion and the vertex.

According to a preferred embodiment the first diodes 4 are 80 in number, while the second diodes 5 are 120 in number.

Advantageously, to further increase the versatility of the patient-specific use, the helmet 1 has a programmable controller with an emission program having programmable values of intensity and duration, and programs for the sequence of emissions and areas for the switching on.

The controller can selectively activate the first diodes 4 and the second diodes 5.

In particular, the switching on of the first 4 and second 5 diodes can be single and individual or synchronous/phased depending on the type of emission.

In particular, depending on the treatment program selected, all LEDs that emit light in the infrared field or, alternatively, all red LEDs or red laser LEDs that emit light in the visible field will light up.

In a preferred embodiment of the invention the controller is programmed with a first program for the simultaneous activation of the first diodes 4 and with a second program for the sequential activation of a part of said first diodes 4 from a central area of the helmet to peripheral areas of the helmet corresponding to lymph drainage areas of the head.

In fact, it should be noted that a part of the LEDs that emit light in the infrared field are activated sequentially, as shown by the arrows indicated in figures 5 and 7, based on a specific program, called DETOX, from the centre of the helmet towards the lateral and rear peripheries of the head, exerting a draining massage towards the lymph drainage routes.

A preferred embodiment of the invention will be described below.

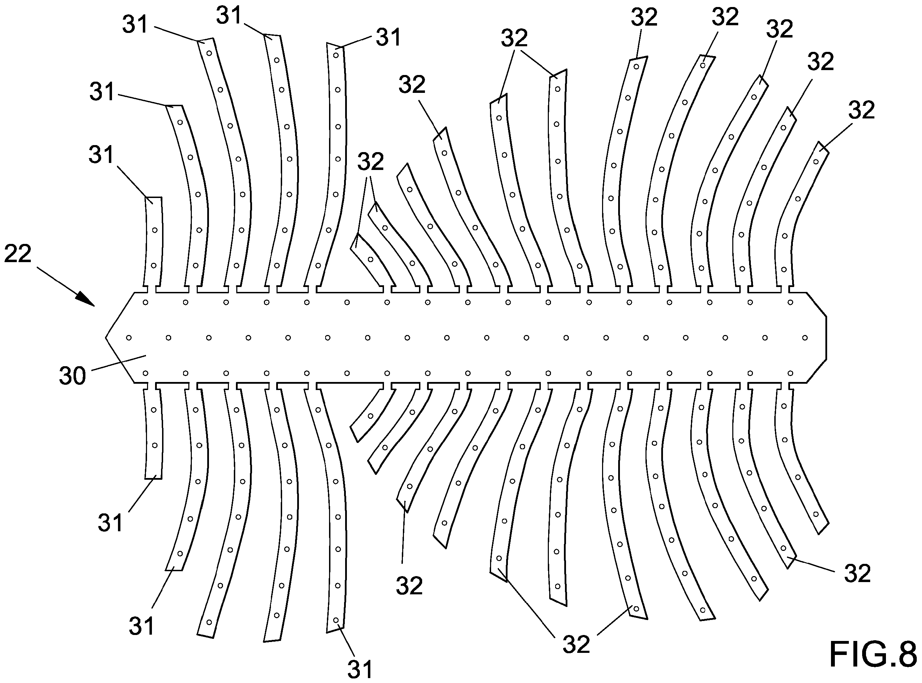

As illustrated in figure 8, said first diodes 4 and said second diodes 5 are integrated on a printed circuit 22 with conformation congruent with the areas of the head subjected to irradiation.

The printed circuit 22 is mounted on a single stiffening support 21 fixed to said shell 2.

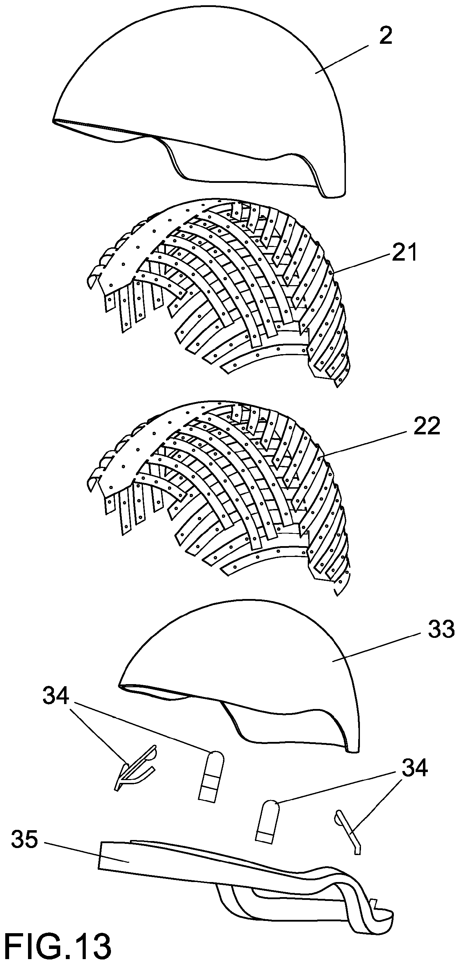

As illustrated in figure 13, the adjustable centring means 6 comprises a spacer 33 with uniform thickness and transparent to the light emitted by the first and second diodes which is applied as a coating of said printed circuit 22, and elastically yieldable centring elements 34 protruding beyond said coating.

The printed circuit 22 is preferably made of flexible material and has a central pin 30 from which lateral branches 31, 32 of different length configured to overlap the areas of the head that must be subjected to irradiation branch bilaterally.

The central pin 30 is positioned in use along the central front-rear direction of the skull.

The branches are divided into a front group 31 of branches that are positioned in use in the front and temple part of the skull and a rear group 32 that is positioned in the parietal, occipital and temporal part of the skull.

The first diodes and the second diodes are positioned both along the central pin and along the lateral branches.

In particular, the printed circuit 22 has a number of stations distributed along the central pin 30 and along the lateral branches 31,32 for the positioning of said first and second diodes.

In particular, all stations comprise the first diodes 4 that emit light in the infrared field.

A part of these stations also comprises the second diodes 5, in particular the LEDs that emit light in the red field.

Only a smaller group of such stations additionally comprises laser LEDs that emit light in the red field.

Therefore, each station can consist of 3 types of circuit:
1) Circuit Type 1: Infrared LED only
2) Circuit type 2: Infrared LED + Red LED
3) Circuit type 3: Infrared LED + red LED + red laser LED.

The type 1 circuit affects the occipital area, while the type 2 and 3 circuits affect the front, parietal and temporal areas.

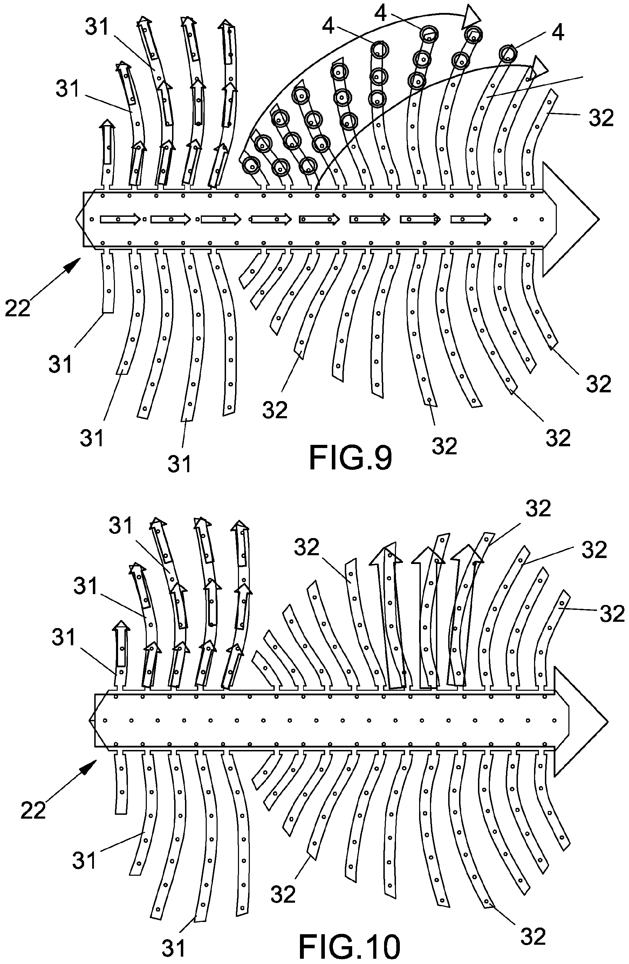

Figures 9 and 10 show two different examples of sequential switching on of a part of said first diodes 4 in the different circuits 1, 2 and 3. The arrows indicate the sequence for the switching on and the directions of the light waves.

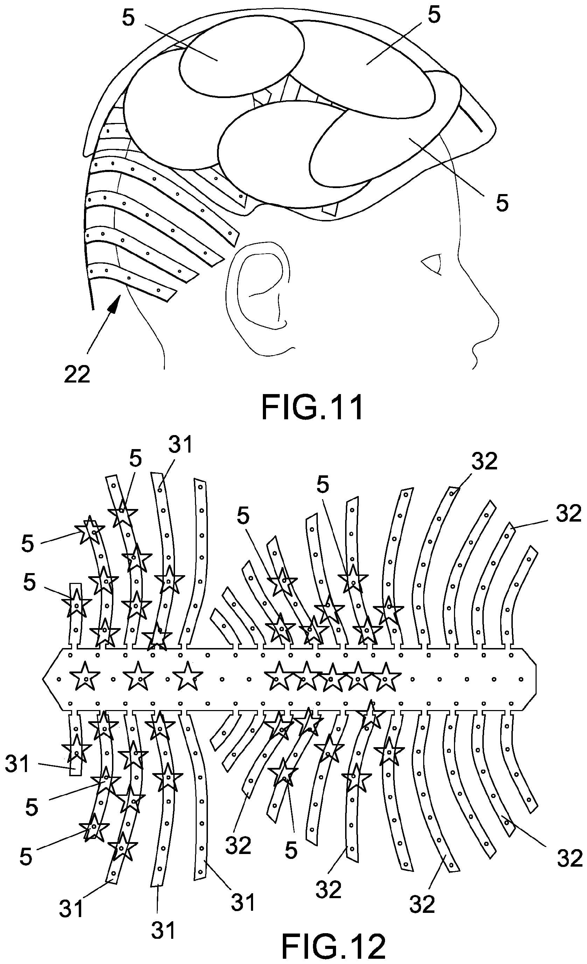

Figure 11 shows the positioning of the red LEDs. In particular, the red LEDs can all be switched on together or the switching on can be customized based on the thinning pattern of the user.

Finally, the helmet 1 has a lower closing element 35 configured to be coupled to said shell 2.

According to a preferred embodiment of the invention the helmet 1 is of the wireless type. This enables the patient to have free hands while using it and enables him/her to be able to conveniently move from one room to another. In this embodiment the helmet 1 further comprises a rechargeable autonomous power supply source.

According to another embodiment of the invention, the helmet 1 has an electrical charging connector that can be connected to the electrical network.

The helmet 1 has an on/off power button through which the user can select the predefined treatment programs.

The predefined programs are as follows:
- DETOX program: duration 10 minutes, only infrared LED activation. The LEDs will light up consecutively starting from the centre of the helmet in 3 different directions, as illustrated by the arrows in figure 4 and 6, and will end up in the 3 lymph drainage points, i.e. behind the ears and on the occipital part.
- CORRECTION/HAIRLOSS program: duration 20 minutes, of which 10 minutes for the activation of the infrared LEDs and 10 minutes for the activation of the LASER LEDs.
- THICKENING/STIMULATION program: duration 20 minutes, of which 5 minutes for the activation of the infrared LEDS and 15 minutes for the activation of the LASER LEDs.

According to the invention, the possibility of uploading a fourth program further customized for the specific user of the helmet is also envisaged.

Once selected, the programs will be visible as a series of light indicators positioned close to the on/off power button will light up. In particular, the light indicators are from 1 to 4 in number.

The helmet 1 has an interface for a wired or wireless connection configured to receive and transmit data.

In particular, it is envisaged realizing an electronic device (for example a smartphone or a PC) presenting an application associated with the helmet that can be consulted both by the patient and by the doctor who prescribed the type of program in order to monitor the correct use of the helmet. In fact, through a relevant program, the application can record the use times, the days and also the programs used.

Through the application it will also be possible to upload software updates, including user-specific/customized programs for the individual user, the result of research and development studies.

The helmet for the treatment of the scalp and hair thus conceived, is susceptible to numerous modifications and variations all falling within the scope of the inventive concept described and claimed.

## Claims

1. A helmet (1) which can be worn on a user's head for the treatment of the scalp and hair with photo-biostimulation technology comprising a shell (2) having first diodes (4) configured for the emission of light in the infrared field and second diodes (5) configured for the emission of light in the visible field and arranged to irradiate the front, parietal, occipital, temporal area and the entire vertex of a user's head, said shell (2) also having adjustable centring means (6) for centring said helmet (1) with respect to the cranial surface of the user, **characterized in that** it has a controller programmed with a first program for the simultaneous activation of said first diodes (4) and with a second program for the sequential activation of a part of said first diodes (4) from a central area of the helmet to peripheral areas of the helmet corresponding to lymph drainage areas of the head.

2. The helmet (1) which can be worn on a user's head for the treatment of the scalp and hair with photo-biostimulation technology according to claim 1, **characterized in that** said second diodes (5) configured to emit light in the visible field are LED diodes and laser diodes.

3. The helmet (1) which can be worn on a user's head for the treatment of the scalp and hair with photo-stimulation technology according to any preceding claim **characterized in that** said first diodes (4) and said second diodes (5) are integrated on a printed circuit (22) with conformation congruent with the areas of the head subjected to irradiation, said printed circuit having a central pin (30) from which lateral branches (31, 32) of different length branch bilaterally.

4. The helmet (1) which can be worn on a user's head for the treatment of the scalp and hair with photo-biostimulation technology according to the preceding claim **characterized in that** said printed circuit (22) is mounted on a single stiffening support (21) fixed to said shell (2).

5. The helmet (1) which can be worn on a user's head for the treatment of the scalp and hair with photo-stimulation technology according to any one of claims 3 and 4, **characterized in that** said central pin 30 is positioned in use along the central front-rear direction of the skull, and said branches are divided into a front group 31 of branches that are positioned in use in the front and temple part of the skull and a rear group 32 that is positioned in the parietal, occipital and temporal part of the skull.

6. The helmet (1) which can be worn on a user's head for the treatment of the scalp and hair with photo-stimulation technology according to any one of claims 3 to 5, **characterized in that** said adjustable centring means (6) comprises a spacer (33) with uniform thickness and transparent to the light emitted by the first and second diodes which is applied as a coating of said printed circuit (22), and elastically yieldable centring elements (34) protruding beyond said coating.

7. The helmet (1) which can be worn on a user's head for the treatment of the scalp and hair with photo-stimulation technology according to any one of claims 1 and 2, **characterized in that** said adjustable centring means (6) comprises movable spacing elements (8) in sliding seats (7).

8. The helmet (1) which can be worn on a user's head for the treatment of the scalp and hair with photo-biostimulation technology according to the preceding claim **characterized in that** said spacing elements (8) are slidable in the front-rear direction of the helmet (1).

9. The helmet (1) which can be worn on a user's head for the treatment of the scalp and hair with photo-stimulation technology according to any one of claims 7 and 8 **characterized in that** said spacing elements (8) are made of silicone material.

10. The helmet (1) which can be worn on a user's head for the treatment of the scalp and hair with photo-biostimulation technology according to any one of claims 7 to 9 **characterized in that** said spacing elements (8) comprise strips (8a) having a series of lower protrusions (8b) distributed at an appropriate pitch along said strips (8a).

11. The helmet (1) which can be worn on a user's head for the treatment of the scalp and hair with photo-biostimulation technology according to any preceding claim **characterized in that** it comprises an interchangeable stabilizing element (12) to stabilize the fit of the helmet (1) as the size of the user's head varies.

12. The helmet (1) which can be worn on a user's head for the treatment of the scalp and hair with photo-biostimulation technology according to any preceding claim **characterized in that** said stabilizing element (12) is a soft element removably housed by mechanical interference in a perimeter seat (13) of the inner side of the helmet shell.

13. The helmet (1) for the treatment of the scalp and hair with photo-biostimulation technology according to claim 12, **characterized in that** said stabilizing element (12) has a tubular conformation.

14. The helmet (1) for the treatment of the scalp and hair with photo-biostimulation technology according to any preceding claim **characterized in that** it has a programmable controller with an emission program having programmable values of intensity, and duration, and programs for the sequence of emissions and areas for the switching on.

## Patentansprüche

1. Helm (1), der auf dem Kopf eines Benutzers für die Behandlung der Kopfhaut und der Haare mit der Technologie der Photobiostimulation getragen werden kann, umfassend eine Schale (2) mit ersten Dioden (4), die für die Emission von Licht im Infrarotbereich konfiguriert sind, und zweiten Dioden (5), die für die Emission von Licht im sichtbaren Bereich konfiguriert sind, und die so angeordnet sind, dass sie den vorderen, parietalen, okzipitalen, temporalen Bereich und den gesamten Scheitel des Kopfes eines Benutzers bestrahlen, wobei die Schale (2) auch einstellbare Zentriermittel (6) aufweist, um den Helm (1) in Bezug auf die Schädeloberfläche des Benutzers zu zentrieren, **dadurch gekennzeichnet, dass** sie eine Steuerung aufweist, die mit einem ersten Programm für die gleichzeitige Aktivierung der ersten Dioden (4) und mit einem zweiten Programm für die sequentielle Aktivierung eines Teils der ersten Dioden (4) von einem zentralen Bereich des Helms zu peripheren Bereichen des Helms, die Lymphdrainagebereichen des Kopfes entsprechen, programmiert ist.

2. Helm (1), der auf dem Kopf eines Benutzers getragen werden kann, für die Behandlung der Kopfhaut und der Haare mit der Technologie der Photobiostimulation nach Anspruch 1, **dadurch gekennzeichnet, dass** die zweiten Dioden (5), die so konfiguriert sind, dass sie Licht im sichtbaren Bereich emittieren, LED-Dioden und Laserdioden sind.

3. Helm (1), der auf dem Kopf eines Benutzers getragen werden kann, für die Behandlung der Kopfhaut und der Haare mit der Photostimulationstechnologie nach einem der vorhergehenden Ansprüche, **dadurch gekennzeichnet, dass** die ersten Dioden (4) und die zweiten Dioden (5) auf einer gedruckten Schaltung (22) integriert sind, deren Form mit den der Bestrahlung unterworfenen Bereichen des Kopfes kongruent ist, wobei die gedruckte Schaltung einen zentralen Stift (30) aufweist, von dem seitliche Zweige (31, 32) unterschiedlicher Länge beidseitig abzweigen.

4. Helm (1), der auf dem Kopf eines Benutzers getragen werden kann, zur Behandlung der Kopfhaut und der Haare mit der Technologie der Photobiostimulation nach dem vorhergehenden Anspruch, **dadurch gekennzeichnet, dass** die gedruckte Schaltung (22) auf einem einzigen Versteifungsträger (21) montiert ist, der an der Schale (2) befestigt ist.

5. Helm (1), der auf dem Kopf eines Benutzers für die Behandlung der Kopfhaut und der Haare mit der Photostimulationstechnologie nach einem der Ansprüche 3 und 4 getragen werden kann, **dadurch gekennzeichnet, dass** der zentrale Stift (30) bei der Verwendung entlang der zentralen Vorder-Hinter-Richtung des Schädels positioniert ist, und die Zweige in eine vordere Gruppe (31) von Zweigen, die bei der Verwendung im vorderen und Schläfenbereich des Schädels positioniert sind, und eine hintere Gruppe (32), die im parietalen, okzipitalen und temporalen Bereich des Schädels positioniert ist, unterteilt sind.

6. Helm (1), der auf dem Kopf eines Benutzers getragen werden kann, zur Behandlung der Kopfhaut und der Haare mit der Photostimulationstechnologie nach einem der Ansprüche 3 bis 5, **dadurch gekennzeichnet, dass** das einstellbare Zentriermittel (6) einen Abstandshalter (33) mit gleichmäßiger Dicke, der für das von der ersten und der zweiten Diode emittierte Licht transparent ist und als Beschichtung der gedruckten Schaltung (22) aufgebracht ist, und elastisch nachgiebige Zentrierelemente (34), die über die Beschichtung hinausragen, umfasst.

7. Helm (1), der auf dem Kopf eines Benutzers für die Behandlung der Kopfhaut und des Haares mit Photostimulationstechnologie nach einem der Ansprüche 1 und 2 getragen werden kann, **dadurch gekennzeichnet, dass** das einstellbare Zentriermittel (6) bewegliche Abstandselemente (8) in Gleitsitzen (7) umfasst.

8. Helm (1), der auf dem Kopf eines Benutzers zur Behandlung der Kopfhaut und der Haare mit der Photobiostimulationstechnologie nach dem vorhergehenden Anspruch getragen werden kann, **dadurch gekennzeichnet, dass** die Abstandselemente (8) in der Vorwärts-Rückwärtsrichtung des Helms (1) verschiebbar sind.

9. Helm (1), der auf dem Kopf eines Benutzers für die Behandlung der Kopfhaut und der Haare mit der Photostimulationstechnologie nach einem der Ansprüche 7 und 8 getragen werden kann, **dadurch gekennzeichnet, dass** die Abstandselemente (8) aus Silikonmaterial hergestellt sind.

10. Helm (1), der auf dem Kopf eines Benutzers getragen werden kann, für die Behandlung der Kopfhaut und der Haare mit der Photobiostimulationstechnologie nach einem der Ansprüche 7 bis 9, **dadurch gekennzeichnet, dass** die Abstandselemente (8) Streifen (8a) mit einer Reihe von unteren Vorsprüngen (8b) umfassen, die in einem geeigneten Abstand entlang der Streifen (8a) verteilt sind.

11. Helm (1), der auf dem Kopf eines Benutzers für die Behandlung der Kopfhaut und der Haare mit der Photobiostimulationstechnologie nach einem der vorhergehenden Ansprüche getragen werden kann, **dadurch gekennzeichnet, dass** er ein austauschbares Stabilisierungselement (12) umfasst, um die Passform des Helms (1) zu stabilisieren, wenn die Größe des Kopfes des Benutzers variiert.

12. Helm (1), der auf dem Kopf eines Benutzers zur Behandlung der Kopfhaut und des Haares mit Photobiostimulationstechnologie nach einem der vorhergehenden Ansprüche getragen werden kann, **dadurch gekennzeichnet, dass** das Stabilisierungselement (12) ein weiches Element ist, das durch mechanische Einwirkung in einem Umfangssitz (13) der Innenseite der Helmschale entfernbar untergebracht ist.

13. Helm (1) für die Behandlung der Kopfhaut und der Haare mit der Technologie der Photobiostimulation nach Anspruch 12, **dadurch gekennzeichnet, dass** das Stabilisierungselement (12) eine röhrenförmige Form aufweist.

14. Helm (1) zur Behandlung der Kopfhaut und der Haare mit Photobiostimulationstechnologie nach einem der vorhergehenden Ansprüche, **dadurch gekennzeichnet, dass** er eine programmierbare Steuerung mit einem Emissionsprogramm mit programmierbaren Werten für Intensität und Dauer sowie Programme für die Abfolge der Emissionen und Bereiche für das Einschalten aufweist.

## Revendications

1. Casque (1) pouvant être porté sur la tête d'un utilisateur pour le traitement du cuir chevelu et des cheveux avec une technologie de photo-biostimulation comprenant une coque (2) avec des premières diodes (4) configurées pour l'émission de lumière dans le domaine infrarouge et des secondes diodes (5) configurées pour l'émission de lumière dans le domaine visible et disposées pour irradier la zone frontale, pariétale, occipitale, temporale et tout le vertex de la tête d'un utilisateur, ladite coque (2) comporte également des moyens de centrage réglables (6) pour centrer ledit casque (1) par rapport à la surface crânienne de l'utilisateur, **caractérisée en ce qu'**elle comporte un contrôleur programmé avec un premier programme pour l'activation simultanée desdites premières diodes (4) et avec un second programme pour l'activation séquentielle d'une partie desdites premières diodes (4) à partir d'une zone centrale du casque vers des zones périphériques du casque correspondant à des zones de drainage lymphatique de la tête.

2. Casque (1) pouvant être porté sur la tête d'un utilisateur pour le traitement du cuir chevelu et des cheveux avec la technologie de photo-biostimulation selon la revendication 1, **caractérisé en ce que** lesdites secondes diodes (5) configurées pour émettre de la lumière dans le champ visible sont des diodes LED et des diodes laser.

3. Casque (1) pouvant être porté sur la tête d'un utilisateur pour le traitement du cuir chevelu et des cheveux avec la technologie de photo-stimulation selon toute revendication précédente **caractérisé en ce que** lesdites premières diodes (4) et lesdites secondes diodes (5) sont intégrées sur un circuit imprimé (22) de conformation congruente aux zones de la tête soumises à l'irradiation, ledit circuit imprimé ayant une broche centrale (30) à partir de laquelle partent bilatéralement des branches latérales (31, 32) de différentes longueurs.

4. Casque (1) pouvant être porté sur la tête d'un utilisateur pour le traitement du cuir chevelu et des cheveux avec la technologie de photo-biostimulation selon la revendication précédente **caractérisé en ce que** ledit circuit imprimé (22) est monté sur un seul support raidisseur (21) fixé à ladite coque (2).

5. Casque (1) pouvant être porté sur la tête d'un utilisateur pour le traitement du cuir chevelu et des cheveux avec la technologie de photo-stimulation selon l'une quelconque des revendications 3 et 4, **caractérisé en ce que** ladite broche centrale (30) est positionnée en utilisation le long de la direction centrale avant-arrière du crâne, et lesdites branches sont divisées en un groupe avant (31) de branches qui sont positionnées en utilisation dans la partie avant et la partie des tempes du crâne et un groupe arrière (32) qui est positionné dans la partie pariétale, occipitale et temporelle du crâne.

6. Casque (1) pouvant être porté sur la tête d'un utilisateur pour le traitement du cuir chevelu et des cheveux par la technologie de photo-stimulation selon l'une quelconque des revendications 3 à 5, **caractérisé en ce que** ledit moyen de centrage réglable (6) comprend une entretoise (33) d'épaisseur uniforme et transparente à la lumière émise par les première et deuxième diodes, qui est appliquée comme revêtement dudit circuit imprimé (22), et des éléments de centrage (34) élastiquement flexibles faisant saillie au-delà dudit revêtement.

7. Casque (1) pouvant être porté sur la tête d'un utilisateur pour le traitement du cuir chevelu et des cheveux par la technologie de photo-stimulation selon l'une quelconque des revendications 1 et 2, **caractérisé en ce que** ledit moyen de centrage réglable (6) comprend des éléments d'espacement mobiles (8) dans des sièges coulissants (7).

8. Casque (1) pouvant être porté sur la tête d'un utilisateur pour le traitement du cuir chevelu et des cheveux avec la technologie de photo-biostimulation selon la revendication précédente, **caractérisé en ce que** les éléments d'espacement (8) sont coulissants dans la direction avant-arrière du casque (1).

9. Casque (1) pouvant être porté sur la tête d'un utilisateur pour le traitement du cuir chevelu et des cheveux par la technologie de photo-stimulation selon l'une quelconque des revendications 7 et 8, **caractérisé par le fait que** lesdits éléments d'espacement (8) sont en silicone.

10. Casque (1) pouvant être porté sur la tête d'un utilisateur pour le traitement du cuir chevelu et des cheveux avec la technologie de photo-biostimulation selon l'une quelconque des revendications 7 à 9, **caractérisé en ce que** lesdits éléments d'espacement (8) comprennent des bandes (8a) ayant une série de saillies inférieures (8b) réparties à un pas approprié le long desdites bandes (8a).

11. Casque (1) pouvant être porté sur la tête d'un utilisateur pour le traitement du cuir chevelu et des cheveux à l'aide de la technologie de photo-biostimulation selon toute revendication précédente **caractérisé en ce qu'**il comprend un élément stabilisateur interchangeable (12) pour stabiliser l'ajustement du casque (1) lorsque la taille de la tête de l'utilisateur varie.

12. Casque (1) pouvant être porté sur la tête d'un utilisateur pour le traitement du cuir chevelu et des cheveux par la technologie de photo-biostimulation selon toute revendication précédente, **caractérisé par le fait que** l'élément stabilisateur (12) est un élément souple logé de manière amovible par interférence mécanique dans un siège périmétrique (13) de la face interne de la coque du casque.

13. Casque (1) pour le traitement du cuir chevelu et des cheveux par la technologie de photo-biostimulation selon la revendication 12, **caractérisé par le fait que** l'élément stabilisateur (12) a une conformation tubulaire.

14. Le casque (1) pour le traitement du cuir chevelu et des cheveux avec la technologie de photo-biostimulation selon toute revendication précédente **caractérisé en ce qu'**il a un contrôleur programmable avec un programme d'émission ayant des valeurs programmables d'intensité et de durée, et des programmes pour la séquence d'émissions et des zones pour l'allumage.
